# EUROPEAN PATENT SPECIFICATION

(11) **EP 2 952 586 B1**
(45) Date of publication and mention of the grant of the patent: **27.10.2021**
(21) Application number: 15170012.7
(22) Date of filing: 01.06.2015
(51) Int. Cl.: C12P 19/00, C12P 19/04

(54) **BACTERIAL CAPSULAR POLYSACCHARIDE YIELD ENHANCEMENT BY ADDITION OF DEFOAMING AGENTS**
ERTRAGSERHÖHUNG VON BAKTERIELLEM KAPSELPOLYSACCHARID DURCH ZUGABE VON ENTSCHÄUMUNGSMITTELN
AMÉLIORATION DU RENDEMENT DE POLYSACCHARIDE CAPSULAIRE BACTÉRIEN PAR ADDITION D'AGENTS ANTIMOUSSE

(30) Priority: 02.06.2014 IN 1821MU2014
(43) Date of publication of application: 09.12.2015
(73) Proprietor: Serum Institute Of India Private Limited, Pune 411 028, Maharashtra (IN)
(72) Inventor: Peddireddy, Srinivas Reddy, IN-411 028 Pune, Maharashtra (IN); Singh, Digamber Chahar, IN-411 028 Pune, Maharashtra (IN); Pisal, Sambhaji Shankar, IN-411 028 Pune, Maharashtra (IN); Dhere, Rajeev Mhalasakant, IN-411 028 Pune, Maharashtra (IN)
(74) Representative: Croce, Valeria

(56) References cited:
- EP-A1- 0 180 664
- WO-A2-2014/080423
- US-A1- 2004 229 319
- US-A1- 2010 297 166
- HOLMES W ET AL: "Evaluation of antifoams in the expression of a recombinant FC fusion protein in shake flask cultures of Saccharomyces cerevisiae & Pichia pastoris", MICROBIAL CELL FACTORIES, BIOMED CENTRAL, LONDON, NL, vol. 5, no. 1, 2006, page P30, XP002512428, ISSN: 1475-2859, DOI: 10.1186/1475-2859-5-S1-P30
- SARAH J ROUTLEDGE: "BEYOND DE-FOAMING: THE EFFECTS OF ANTIFOAMS ON BIOPROCESS PRODUCTIVITY", COMPUTATIONAL AND STRUCTURAL BIOTECHNOLOGY JOURNAL, vol. 3, no. 4, October 2012 (2012-10), pages 1-7, XP055218287, ISSN: 2001-0370, DOI: 10.5936/csbj.201210014
- JUNKER BETH: "Foam and its mitigation in fermentation systems", BIOTECHNOLOGY PROGRESS, AMERICAN INSTITUTE OF CHEMICAL ENGINEERS, US, vol. 23, no. 4, 2007, pages 767-784, XP002681058, ISSN: 8756-7938, DOI: 10.1021/BP070032R [retrieved on 2008-09-05]
- None

## Description

Neisseria meningitidis is the cause of epidemic bacterial meningitis. Capsular polysaccharide is a major virulence determinant of N. meningitidis. Among the 13 meningococcal serogroups classified based on capsular polysaccharide structure, serogroups A, B, C, Y, and W135 are associated with the majority of cases of meningococcal disease. In the African meningitis belt most large epidemics have been caused by serogroup A meningococci, whereas sporadic disease and outbreaks in developed countries are usually caused by serogroup B and C meningococci. Serogroup Y meningococci emerged as an important cause of sporadic disease and outbreaks in the United States in the late 1990s , and in 2000 serogroup W135 meningococci caused worldwide disease in association with the Hajj pilgrimage and large outbreaks in sub-Saharan Africa. Further Serogroup X Neisseria meningitidis (MenX), previously a rare cause of sporadic cases of meningitis, has recently been associated with increased incidence of meningococcal disease and has emerged as a cause of large outbreaks in the "Meningitis Belt" of Africa.

The capsular polysaccharides of serogroup B, C, Y, and W135 meningococci are composed of sialic acid derivatives. Serogroup B and C meningococci express (α 2-8)- and ( α 2- 9) - linked polysialic acid, respectively, while alternating sequences of D-glucose or D-galactose and sialic acid are expressed by serogroup Y and W135 N. meningitidis. In contrast, the capsule of serogroup A meningococci is composed of (α 1- 6)-linked N-acetylmannosamine 6-phosphate , while N. meningitidis serogroup X synthesizes capsular polymers of ( α 1 -4)-linked N-acetylglucosamine 1-phosphate. The MenX PS structure is a homopolymer of 1→4-linked N-acetyl-d-glucosamine 1-phosphate. The conjugation of meningococcal capsular polysaccharides to a carrier protein has led to the development of monovalent (A or C) polysaccharide conjugate vaccines with high effectiveness, and immunogenicity data from clinical trials indicate that wide use of tetravalent and pentavalent conjugate vaccines covering serogroups A, C, Y ,W-135 and X may be similarly effective. Several synthetic media were discovered for large-scale production of meningococcal polysaccharide (Frantz, I. D. Jr. Growth Requirements of the Meningococcus. J. Bact., 43: 757-761, 1942; Catlin, B. W. Nutritional profiles of Neisseria lactamica, gonorrhoeae and meningitidis, in chemically defined media. J. Inf. Dis., 128(2): 178-194, 1973; R. Watson et al., "The specific hapten of group C (group IIa) meningococcus, II. Chemical nature", J Immunol, 81 (4),337-344, April 1958; M. Fossa da Paz et al., "Polysaccharide production in batch process of Neisseria meningitidis serogroup C comparing Frantz, modified Frantz and Catlin 6 cultivation media", Braz. J. Microbiol. vol. 34., no. 1., pp. 27-32, January/April 2003). U.S. Pat. No. 5,494,808 reports a large-scale, high-cell density (5 g/L dry cell weight, and an optical density of between about 10-13 at 600 nm) fermentation process for the cultivation of N. meningitidis(serogroup B 11). This patent disclose the following medium (called "MC.6") for culturing Neisseria meningitidis for isolation of OMPC ("Outer Membrane Protein Complex")

US 7399615 discloses a fermentation composition wherein the composition omits NH4Cl, and an improved method of fermenting Neisseria (serogroups A,C,Y & W135) in a fermentation composition replaces ammonium chloride (nitrogen source) with a soy peptone (HSP-A; Nutricepts, Inc; Minneapolis, Minn.) . The said fed batch fermentation (2L), wherein the fermentation medium as well as feed solution contains HSP-A results in Men A polysaccharide yield at fermentation harvest stage of about 1.3 to 1.4 g/L at an average max OD between 14-20.

US 7491517 discloses a Neisseria meningitidis fastidious culture medium (NMFM) for producing capsular polysaccharides from Neisseria meningitides (serogroups A,C,Y & W135) comprising: DI (deionizer) water, NaCl, K2SO4, KCl, trisodium citrate.2H2O, MgSO4.7H2O, MnSO4.H2O, MnCl2.6H2O, vitamin B12, NAD (Nicotinamide Adenine Dinucleotide) thiamine HCl, soy peptone, D-glucose, L-glutamic acid, L-arginine, L-serine, L-cysteine, glycine, morpholinepropanesulphonic acid [MOPS], CaC03 to maintain pH at 6.5 to 7.0 and Fe2(SO4)3 for serogroup A and NH4Cl for serogroup W-135.The said fermentation results in polysaccharide yield of about 30-40 mg/L at an average max OD of 10.

D. Bundle et al., "Studies on the group-specific polysaccharide of Neisseria meningitidis serogroup X and an improved procedure for its isolation", J Biol Chem. 249(15), 4797-801, Aug 1974 discusses preparation and isolation of Men X polysaccharide from N.meningitidis strain 247 X (Laboratory Center for Disease Control) wherein said strain was grown on a chemically defined medium (NCDM) for 18 hours. The said fermentation results in Men X polysaccharide yield of about 20 mg/L. Micoli et al (WO2013/174832) discloses a Modified Frantz fermentation medium comprising of L-glutamic acid (1.6 g/L), Na2HPO4·2H2O (15.5 g/L), KCl (0.09 g/L), NH4Cl (1.25 g/L), pH 7.6, supplemented with glucose (50 g/L), MgSO4·7H2O (30 g/L), 25g/L ultrafiltered yeast extract, L-cysteine(1.5 g/L)that results in 356 mg/L N.meningitidis X polysaccharide at fermentation harvest stage.

Sub-Saharan belt is the region that is the primary market for Meningococcal conjugate vaccines. Having said that, cost-effective manufacturing processes for such vaccines have become a priority. Increasing capsular polysaccharide "fermentation harvest stage yield" by employing novel feed strategies and fermentation medium has been one of the preferable approach to achieve said objective. However all the available fermentation processes previously disclosed fail to improve "fermentation harvest stage yield" of N.meningitidis capsular polysaccharide beyond 1.5 g/L. Also formation of foam during large scale fermentation could i) reduce capsular polysaccharide yield due to loss of cells and culture medium to the foam phase, ii) can be detrimental to cells since when bubbles burst they exert sheer forces iii) result in a loss of sterility if the foam escapes and iv) can lead to over-pressure if a foam-out blocks an exit filter.

To prevent the formation of foam, mechanical foam breakers, ultrasound or, most often, the addition of chemical antifoaming agents (or "antifoams") are routinely employed. Foams are classified as either hydrophobic solids dispersed in carrier oil, aqueous suspensions/emulsions, liquid single components or solids.

Several mechanisms of action for these agents have been suggested which include bridging-dewetting, spreading fluid entrainment and bridging-stretching. Many are commercially-available, with 19 being sold by Sigma-Aldrich alone. While little information is routinely given about their composition, their specific antifoam properties have been thoroughly investigated. These include their effects on foam height with time, their influence on the volumetric oxygen mass transfer coefficient (kLa) of the system, their gas hold-up characteristics and their globule size and distribution in relation to their action upon foams . Such studies have been performed in various growth media in both the absence and presence of cultures of prokaryotic and eukaryotic microbes. It is known that certain antifoams can affect the growth rates of both prokaryotic and eukaryotic organisms in addition to changing surface properties such as lipid content, resulting in changes to permeability. This in turn can be beneficial to a recombinant protein production system for soluble proteins, as has been demonstrated by increased secretion of α-amylase and GFP. Reference is made to S.J. Routledge "Beyond de-foaming: the effects of antifoams on bioprocess productivity" Computational and Structural Biotechnology Journal, 3(4), 1-7, Oct 2012. To evaluate their potential to enhance fermentation performance, vegetable oils were investigated in a model tetracycline fermentation. With sucrose as the carbon source, the fermentation efficiency of Streptomyces aureofaciens (ATCC 10762) was enhanced by the inclusion in the medium of low levels of vegetable oil. Soybean and sunflower oils significantly improved the rate of sucrose consumption and tetracycline production suggesting that oil is an excellent adjuvant for improving fermentation productivity. Reference is made to A.M. Jones, M. A. Porter "Vegetable oils in fermentation: beneficial effects of low-level supplementation" J Ind Microbiology and Biotechnology, 121(4), 203-207, Oct 1998.

A nutritional food rich in menaquinone-7 has a potential in preventing osteoporosis and cardiovascular diseases. The major issues for the bulk production of menaquinone-7 are the low fermentation yield, biofilm formation and the use of organic solvents for the vitamin extraction. Further dynamic fermentation involving use of vegetable oil as antifoam, high stirring and aeration rates enhances the yield of fermentation process significantly compared to static system. Reference is made to A. Berenjian et al., " Designing of an Intensification Process for Biosynthesis and Recovery of Menaquinone-7" Applied Biochemistry and Biotechnology, 172, 1347-1357, 2014. It has been disclosed earlier that Sonit (an antifoaming agent derived from hydrogenated vegetable oil)was especially effective in increasing biomass and penicillin yields from Penicillium chrysogenum. Reference is made to M. Gavrilescu "Research on the behavior of some antifoam agent during penicillin biosynthesis" Revista de Chimie 40, 388-391, 1989.

Use of 300% Dow 1520 antifoam solution to control foaming during N.meningitidis A , C,W135,Y fermentations has been previously reported. Reference is made to US 20080274515. However, the present invention arises from a surprising finding that an antifoam in the form of alkoxylated fatty acid esters on a vegetable bases, when added gradually along with optimal feed solution comprising of advantageous combination of carbon and nitrogen sources during log phase was found to increase the optical density(OD₅₉₀) and thereby enhanced "N.meningitidis Serogroup A capsular polysaccharide" yield to above 2 g/L at fermentation harvest stage.

Although defoaming agents have been reported for enhancing yields of recombinant proteins in prokaryotic and eukaryotic systems, none of the prior art has examined antifoams that are conventionally used in controlling the foaming of bacterial fermentation cultures for their effects over and above that of their de-foaming action such as effect of antifoam on the yield of capsular polysaccharides in cultures of N.meningitidis.

### Summary of Invention:

According to present invention gradual addition of a 10%v/v to 20% v/v solution of an antifoam, being an alkoxylated fatty acid ester on a vegetable base added at a final concentration from 0.01% to 0.1% to *Neisseria meningitides A* cultures from 4^{th} hr of log phase onwards till 11^{th} hr i) increase the OD₅₉₀ by 5 units, thereby obtaining OD₅₉₀ of about 22 to 27 in comparison to OD₅₉₀ of about 15 to 17 observed for fermentation cultures without this antifoam, ii) extends the duration of log phase iii) thereby obtaining significantly improved capsular polysaccharide yield of about 2 g/L to 2.5 g/L at fermentation harvest stage such that the polysaccharide at fermentation harvest stage has minimum load of contaminants like protein and nucleic acid.

### Description of the figures

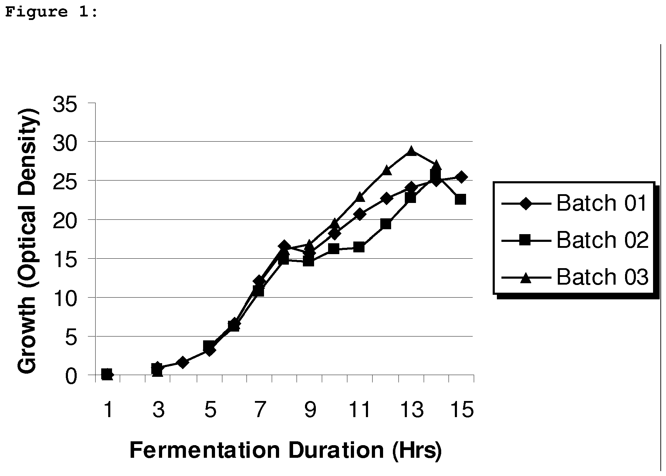

**Figure 1****:** Men A Growth profile at 20 L scale - Gradual addition of Struktol J673A® results in an increase in Optical Density (OD) thereby extending log phase.

### Detailed Description

The present invention provides significant improvement in *Neisseria meningitidis serogroup A* capsular polysaccharide yield at fermentation harvest stage by gradual addition of an antifoam agent alongwith an optimal feed solution from log phase onwards.

Preferably, said antifoam can enhance yield of N.meningitidis serogroup A capsular polysaccharide wherein i) said antifoam could have altered the permeability of membrane which results in increased and efficient uptake of medium components and ii)alkoxylated fatty acid ester or the vegetable base of antifoam could have been metabolized as an additional nutrient source.

An important aspect of the instant invention is that said antifoam agent is an alkoxylated fatty acid ester on a vegetable base.

An example of said antifoam agent is Struktol J673A which is an alkoxylated fatty acid ester on a vegetable base and is used as a 10%v/v to 20% v/v solution. It is added at a final concentration in fermenter from 0.01% to 0.1% v/v.

The N.meningitidis A polysaccharide yield at fermenter harvest stage can be at least 2 fold greater than the yield reported in prior art.

The process of the present invention is applied to produce N.meningitidis serotype A capsular polysaccharide, more preferably to "Men A strain M1027". The process could also be utilized for N.meningitidis serogroups selected from B,C D, Y, Z, 29E and W-135.

The fermentation medium used in the process of the invention having pH from 7 to 7.1 , comprises dextrose from 8 g/L to 10 g/L, sodium chloride from 5.6 g/L to 6 g/L, dipotassium sulfate from 0.8 g/L to 1 g/L, arginine from 0.2 g/L to 0.4 g/L, serine from 0.4 g/L to 0.6 g/L, cysteine from 0.20 g/L to 0.25 g/L, magnesium chloride (MgCl2) from 0.18 g/L to 0.20 g/L, calcium chloride (CaCl₂) from 0.020 g/L to 0.022 g/L, ferrous sulphate (FeSO₄) at 0.002 g/L, L-glutamic acid from 4.8 g/L to 5.2 g/L, soya peptone from 8.8 g/L to 9.2 g/L, ammonium chloride (NH₄Cl) from 0.44 g/L to 0.46 g/L, di-potassium hydrogen phosphate (K₂HPO₄) from 3.8 g/L to 4.2 g/L and yeast extract from 5.30 g/L to 5.35 g/L. Preferably said fermentation medium of used for the fermentation method of the present invention can comprise dextrose at 10 g/L, sodium chloride at 5.8 g/L, dipotassium sulphate at 1 g/L, arginine at 0.3 g/L, serine at 0.5 g/L, cysteine at 0.23 g/L, magnesium chloride (MgCl₂) at 0.19 g/L, calcium chloride (CaCl₂) at 0.021 g/L, ferrous sulphate (FeSO₄) at 0.002 g/L, L-glutamic acid at g/L, soya peptone at 9 g/L, ammonium chloride (NH₄Cl) at 0.45 g/L, dipotassium hydrogen phosphate (K₂HPO₄) at 4 g/L and yeast extract at 5.34 g/L. The fermentation method of the present invention uses a feed solution comprising dextrose from 70 g/L to 75 g/L, mono sodium glutamate from 37 g/L to 37.5 g/L, arginine from 2.8 g/L to 3.2 g/L, serine from 2.8 g/L to 3.2 g/L, magnesium chloride (MgCl₂) from 1.8 g/L to 2.2 g/L, calcium chloride (CaCl₂) from 0.13 g/L to 0.15 g/L, ferrous sulphate (FeSO₄) at 0.02 g/L, soya peptone from 4.8 g/L to 5.2 g/L, cysteine from 1.8 g/L to 2.2 g/L and yeast extract from 8.2 g/L to 8.4 g/L. Preferably said feed solution of present invention comprises dextrose at 75 g/L, mono sodium glutamate at 37.5 g/L, arginine at 3.0 g/L, serine at 3.2 g/L, magnesium chloride (MgCl₂) from 1.8 g/L to 2.2 g/L, calcium chloride (CaCl₂) from 0.13 g/L to 0.15 g/L, ferrous sulphate (FeSO₄) at 0.02 g/L, soya peptone at 5 g/L, cysteine at 2.0 g/L and yeast extract at 8.3 g/L. An important aspect of present invention is that antifoam is gradually added as a 10%v/v to 20% v/v solution at a final concentration from 0.01% to 0.1% to *N*. *meningitides A* cultures from 4^{th} hr of log phase till 11^{th} hr thereby resulting in i) an OD₅₉₀ of about 22 to 27 in comparison to OD₅₉₀ of about 15 to 17 observed for other antifoams, ii) extended duration of log phase iii)minimum level of protein, nucleic acid contaminants. Preferably said antifoam addition can begin at 4^{th} hr of fermentation at a rate of about 20 ml /hr initially and thereafter at a rate of about 35- 50 ml /hr.

The instant invention provides a novel fermentation method for obtaining higher N.meningitidis serogroup A capsular polysaccharide yield of about 2 to 2.5 gm/L at fermentation harvest stage by gradual addition of an antifoam agent alongwith an optimal feed solution from log phase onwards, characterized in that an antifoam comprising of alkoxylated fatty acid ester on a vegetable base is metabolized as a nutrient by N.meningitidis thereby providing at least 2 fold polysaccharide yield improvement as compared to i) yield observed for fermentation cultures without this antifoam or ii) yield observed for fermentation cultures employing polyalkylene glycol based surfactants as antifoam.

### Examples

### Fermentation Procedure

Seed vial containing 3 ml of "N.meningitidis A - strain M1027"(NIH) culture having OD 1/ml was freezed at -70°C. Then vial was thawed and seeded into 40 ml of seed medium which was incubated at 37 °C and agitated at 150 to 180 rpm. The 40 ml seed culture with OD > 1.0 ± 0.2 was directly inoculated to 800 ml seed medium and the culture was again incubated at 37 °C under agitation 160 -200 rpm, till the OD reached > 1.0 ± 0.2. Seed Culture having OD 1.0 ± 0.2 with volume 800 ml was seeded into the reactor, wherein the fermentation medium volume in the reactor was ~ 12L- 14L. After inoculation of reactor 0 hr OD was maintained at 0.04 to 0.06.pH during the fermentation process was maintained around 7.1.The entire fermentation process was carried out in a 30L Stainless steel bioreactor, wherein fermentation cycle was run in a continuous fed batch mode and total duration of fermentation cycle was around 12-18hrs.

### Harvesting & Inactivation

The fermentation process was terminated once drop in optical density was observed followed by inactivation using 1-2% formaldehyde for about 2hrs to 3hrs at 37 °C. Further the temperature was reduced to 10 °C and incubated for 30 minutes. The harvest was unloaded and centrifuged at 14,500g for 45-60 minutes the inactivated cell pellet was discarded and the supernatant was subjected to 0.2u sterile filtration followed by 100KD diafiltration (10-15 times) and is further concentrated with WFI.

**Table 1:Men A Polysaccharide Characteristics at fermentation harvest stage observed for Breox® and Struktol J673A®**

| | **Antifoam -Breox®** | **Antifoam -Struktol J673A®** |
|---|---|---|
| **Men A Polysaccharide Yield mg/1 (Fermentation harvest stage)** | 750mg/L to 1100 mg/L | 2gm/L to 2.5gm /L |
| **Impurity (content of Nucleic acid and Protein) post- fermentation harvest** | Protein: 600 mg/L to 900 mg/L | Protein :750 mg/L to 900mg/L |
| | Nucleic acid:200 mg/L to 300 mg/L | Nucleic acid: 250mg/L to 750 mg/L of harvest |
| **Size of Harvest Polysaccharide** | 900 KDa to 1400 KDa | 800 KDa to 1200 KDa |

The novel fermentation method utilizing Struktol **J673A®** as antifoam alongwith an optimal fermentation medium and feed solution was found to provide higher N.meningitidis A capsular polysaccharide yield of about 2 to 2.5 g/L at fermentation harvest stage ,thereby providing at least 2 fold polysaccharide yield improvement as compared to polyalkylene glycol based surfactant based antifoam agents like Breox®.

### Purification of Men A polysaccharide

A mixture of 1% deoxycholate, 6% sodium acetate, 2mM EDTA & 40% ethanol was added to the 100KD diafiltered harvest. Then the mixture was kept at 2―8°C for 3-4hrs with stirring. Later mixture was subjected to centrifugation at 10000 rpm for 20min and supernatant was diafiltered against 25 mM Tris with 100KD cassette membrane. Further 3% w/v cetyltrimethylammonium precipitation was carried overnight at 2―8°C with stirring and pellet was collected. Said pellet was dissolved in 96% ethanol with 0.05M NaCl at 2―8°C for 2 hrs on stirring. Then polysaccharide precipitation was carried for 30 minutes and pellet was collected. Said pellet was dissolved in 45% ethanol with 0.4M NaCl for 1hr. The supernatant was collected & filtered through CUNO R32SP carbon filter. Then polysaccharide was precipitated in 96 % ethanol for 1-2 hrs and pellet was collected. Then pellet was dissolved in WFI followed by TFF. Final purified N.meningitidis A polysaccharide was stored at -20° C.

The purified N.meningitidis A polysaccharide prepared by was tested for impurities like proteins, Nucleic acid , Endotoxin and Molecular size .Purified Men A polysaccharide was found to meet WHO specification / guidelines for Men A polysaccharide. Refer Table 2.

**Table 2: Purified Men A polysaccharide-Specifications**

| | | **Batch No. & Results** | |
|---|---|---|---|
| **Parameters & Tests** | **Specification** | | |
| | | **Batch No 001** | **Batch No 002** |
| Polysaccharide recovery | Total mg | 16.536 | 13.839 |
| | | | |
| Polysaccharide content | The total phosphorus content should be not less than 8.0% of the dry weight of isolated product | 9.33 | 9.46 |
| O-Acetyl content | O-Acetylated polysaccharide should not be less than 2mMol/g polysaccharide | 2.63 | 2.68 |
| Protein Impurity | NMT 1% by weight of polysaccharide. | < 0.3 | < 0.3 |
| Nucleic Acid Impurity | NMT 1% by weight of polysaccharide. | 0.07 | 0.07 |
| Endotoxin | Less than 100 IU of endotoxin per µg of polysaccharide. | < 10 | 14.825 |
| Molecular size distribution | At least 65% of polysaccharide is eluted before a distribution coefficient (K_{D})of 0.50 is reached | 78.58 | 78.89 |

Subsequent to purification, Men A Polysaccharide was found to comply with WHO specifications wherein said polysaccharide contained less than 0.3% proteins/peptides, less than 0.07% nucleic acids and less than 10EU/µg endotoxins.

## Claims

1. A fermentation method for obtaining *Neisseria meningitidis serogroup A* capsular polysaccharide comprising:
a) inoculating *Neisseria meningitidis A* into a fermentation medium having a pH between 7 and 7.5 and comprising: Dextrose from 8 g/L to 10 g/L, Sodium Chloride from 5.6 g/L to 6 g/L, Di potassium Sulphate from 0.8 g/L to 1 g/L, Arginine from 0.2 g/L to 0.4 g/L, Serine from 0.4 g/L to 0.6 g/L, Cysteine from 0.20 g/L to 0.25 g/L, Magnesium Chloride (MgCl2) from 0.18 g/L to 0.20 g/L, Calcium Chloride (CaCl2)from 0.020 g/L to 0.022 g/L, Ferrous Sulphate (FeSO₄) at 0.002 g/L, L-Glutamic Acid from 4.8 g/L to 5.2 g/L, Soya peptone from 8.8 g/L to 9.2 g/L, Ammonium Chloride (NH4Cl) from 0.44 g/L to 0.46 g/L, Di-potassium Hydrogen Phosphate (K2HPO4) from 3.8 g/L to 4.2 g/L and Yeast Extract from 5.30 g/L to 5.35 g/L;
b) adding, from log phase onwards, a feed solution comprising: Dextrose from 70 g/L to 75 g/L, Mono sodium Glutamate from 37 g/L to 37.5 g/L, Arginine from 2.8 g/L to 3.2 g/L, Serine from 2.8 g/L to 3.2 g/L, Magnesium Chloride (MgCl2) from 1.8 g/L to 2.2 g/L, Calcium Chloride (CaCl2) from 0.13 g/L to 0.15 g/L, Ferrous Sulphate (FeSO₄) at 0.02 g/L, Soya peptone from 4.8 g/L to 5.2 g/L, Cysteine from 1.8 g/L to 2.2 g/L and Yeast Extract from 8.2 g/L to 8.4 g/L;
c) adding an antifoam agent which is a alkoxylated fatty acid ester on vegetable base as a 10% v/v to 20% v/v solution, starting at 4 hour of fermentation till 11 hour, wherein final concentration of Struktol **J673A®**in fermenter is comprised between 0.01% and 0.1% v/v;
d) harvesting and collecting 2 g/L to 2.5 g/L of *Neisseria meningitidis* A capsular polysaccharide.

2. The method according to claim 1 further comprising, after said step d) a purification step comprising:
- adding a mixture of 1% deoxycholate, 6% sodium acetate, 2mM EDTA and 40% ethanol to 100KD diafiltered harvested *Neisseria meningitidis* A capsular polysaccharide;
- Stirring said mixture at 2―8°C for 3-4 hrs;
- centrifuging at 10000 rpm for 20 minutes said mixture and subjecting the supernatant collected to diafiltration against 25 mM Tris with 100KD cassette membrane;
- precipitating with 3% w/v cetyltrimethylammonium overnight at 2―8°C with stirring;
- collecting the pellet and dissolving the same in 96% ethanol with 0.05M NaCl at 2―8°C for 2 hrs on stirring;
- precipitating for 30 minutes and re-dissolving the pellet in 45% ethanol with 0.4M NaCl for 1hr;
- collecting the supernatant and filtering through carbon filter;
- precipitating in 96 % ethanol for 1-2 hrs and collecting the pellet which is purified *N.meningitidis* A polysaccharide.

3. The method according to claim 1 or 2, wherein said antifoam addition begins at start of Log phase, about 4^{th} hour of fermentation, at a rate of about 20 ml/hr, and thereafter continues at a rate of about 35-50 ml /hr till the end of the Log phase, about 11^{th}― 14^{th} hours of fermentation.

4. The method according to claim 2 or 3, wherein said polysaccharide has a weight average molecular weight from 800 to 1200 KDa, and said purified polysaccharide contains less than 0.3% w/w proteins/peptides, less than 0.07% w/w nucleic acids and less than 10EU/µg endotoxins.

5. The method according to claim 1, wherein said fermentation medium comprises: Dextrose at 10 g/L, Sodium Chloride at 5.8 g/L, Di potassium Sulphate at 1 g/L, Arginine at 0.3 g/L, Serine at 0.5 g/L, Cysteine at 0.23 g/L, Magnesium Chloride (MgCl₂) at 0.19 g/L, Calcium Chloride (CaCl₂) at 0.021 g/L, Ferrous Sulphate (FeSO₄) at 0.002 g/L, L-Glutamic Acid at 5 g/L, Soya peptone at 9 g/L , Ammonium Chloride (NH₄Cl) at 0.45 g/L, Di-potassium Hydrogen Phosphate (K₂HPO₄) at 4 g/L and Yeast Extract at 5.34 g/L.

6. The method according to claim 1, wherein said feed solution comprises: Dextrose at 75 g/L , Mono sodium Glutamate at 37.5 g/L, Arginine at 3.0 g/L, Serine at 3.2 g/L, Magnesium Chloride (MgCl₂) from 1.8 g/L to 2.2 g/L, Calcium Chloride (CaCl₂) from 0.13 g/L to 0.15 gm/L, Ferrous Sulphate (FeSO₄) at 0.02 g/L, Soya peptone at 5 g/L, Cysteine at 2.0 g/L and Yeast Extract at 8.3 g/L.

## Patentansprüche

1. Fermentationsverfahren zur Gewinnung von *Neisseria meningitidis Serogruppe A* Kapselpolysaccharid, umfassend:
a) Einimpfen von *Neisseria meningitidis A* in ein Fermentationsmedium mit einem pH-Wert zwischen 7 und 7,5 und umfassend: Dextrose von 8 g/L bis 10 g/L, Natriumchlorid von 5,6 g/L bis 6 g/L, Di-Kaliumsulfat von 0,8 g/L bis 1 g/L, Arginin von 0,2 g/L bis 0,4 g/L, Serin von 0,4 g/L bis 0,6 g/L, Cystein von 0,20 g/L bis 0,25 g/L, Magnesiumchlorid (MgCl2) von 0,18 g/L bis 0,20 g/L, Calciumchlorid (CaCl2) von 0,020 g/L bis 0,022 g/L, Eisensulfat (FeSO₄) zu 0,002 g/L, L-Glutaminsäure von 4,8 g/L bis 5,2 g/L, Sojapepton von 8,8 g/L bis 9,2 g/L, Ammoniumchlorid (NH4Cl) von 0,44 g/L bis 0,46 g/L, Di-Kaliumhydrogenphosphat (K2HPO4) von 3,8 g/L bis 4,2 g/L und Hefeextrakt von 5,30 g/L bis 5,35 g/L;
b) Zugeben, ab der Log-Phase, von einer Ausgangsmateriallösung, umfassend: Dextrose von 70 g/L bis 75 g/L, Mononatriumglutamat von 37 g/L bis 37,5 g/L, Arginin von 2,8 g/L bis 3,2 g/L, Serin von 2,8 g/L bis 3,2 g/L, Magnesiumchlorid (MgCl2) von 1,8 g/L bis 2,2 g/L, Calciumchlorid (CaCl2) von 0,13 g/L bis 0,15 g/L, Eisensulfat (FeSO4) zu 0,02 g/L, Sojapepton von 4,8 g/L bis 5,2 g/L, Cystein von 1,8 g/L bis 2,2 g/L und Hefeextrakt von 8,2 g/L bis 8,4 g/L;
c) Zugeben von einem Antischaummittel, bei dem es sich um einen alkoxylierten Fettsäureester auf pflanzlicher Basis handelt, als eine 10 % v/v bis 20 % v/v Lösung, beginnend bei 4 Stunden Fermentation bis 11 Stunden, wobei die Endkonzentration von Struktol **J673A®**im Fermenter zwischen 0,01 % und 0,1 % v/v liegt;
d) Ernten und Sammeln von 2 g/L bis 2,5 g/L von *Neisseria meningitidis A* Kapselpolysaccharid.

2. Verfahren nach Anspruch 1, ferner umfassend einen Reinigungsschritt nach dem Schritt d), umfassend:
- Zugeben einer Mischung aus 1 % Deoxycholat, 6 % Natriumacetat, 2 mM EDTA und 40 % Ethanol zu 100 KD diafiltriertem geerntetem *Neisseria meningitidis A* Kapselpolysaccharid ;
- Rühren der Mischung bei 2 - 8 °C für 3 - 4 Stunden;
- Zentrifugieren der Mischung bei 10000 U/min für 20 Minuten und Diafiltration des gesammelten Überstandes gegen 25 mM Tris mit 100 KD Kassettenmembran;
- Ausfällen mit 3 % w/v Cetyltrimethylammonium über Nacht bei 2 - 8 °C unter Rühren;
- Sammeln des Pellets und Auflösen desselben in 96 %-igem Ethanol mit 0,05 M NaCl bei 2 - 8 °C für 2 Stunden unter Rühren;
- Ausfällen für 30 Minuten und erneutes Auflösen des Pellets in 45 %-igem Ethanol mit 0,4 M NaCl für 1 Stunde;
- Sammeln des Überstandes und Filtrieren durch Kohlefilter;
- Ausfällen in 96 %-igem Ethanol für 1 bis 2 Stunden und Sammeln des Pellets, das gereinigtes *N. meningitidis A* Polysaccharid ist.

3. Verfahren nach Anspruch 1 oder 2, wobei die Zugabe von Antischaummittel zu Beginn der Log-Phase, etwa in der vierten Stunde der Fermentation, mit einer Rate von etwa 20 ml/h beginnt und danach mit einer Rate von etwa 35 - 50 ml/h bis zum Ende der Log-Phase, etwa in der 11. bis 14. Stunde der Fermentation, fortgesetzt wird.

4. Verfahren nach Anspruch 2 oder 3, wobei das Polysaccharid ein gewichtsmittleres Molekulargewicht von 800 bis 1200 KDa aufweist und das gereinigte Polysaccharid weniger als 0,3 Gew.-% Proteine/Peptide, weniger als 0,07 Gew.-% Nukleinsäuren und weniger als 10 EU/µg Endotoxine enthält.

5. Verfahren nach Anspruch 1, wobei das Fermentationsmedium umfasst: Dextrose zu 10 g/L, Natriumchlorid zu 5,8 g/L, Di-Kaliumsulfat zu 1 g/L, Arginin zu 0,3 g/L, Serin zu 0,5 g/L, Cystein zu 0,23 g/L, Magnesiumchlorid (MgCl2) zu 0,19 g/L, Calciumchlorid (CaCl₂) zu 0,021 g/L, Eisensulfat (FeSO₄) zu 0,002 g/L, L-Glutaminsäure zu 5 g/L, Sojapepton zu 9 g/L, Ammoniumchlorid (NH₄Cl) zu 0,45 g/L, Di-Kaliumhydrogenphosphat (K₂HPO₄) zu 4 g/L und Hefeextrakt zu 5,34 g/L.

6. Verfahren nach Anspruch 1, wobei die Ausgangsmateriallösung Folgendes umfasst: Dextrose zu 75 g/L, Mononatriumglutamat zu 37,5 g/L, Arginin zu 3,0 g/L, Serin zu 3,2 g/L, Magnesiumchlorid (MgCl₂) von 1,8 g/L bis 2,2 g/L, Calciumchlorid (CaCl₂) von 0,13 g/L bis 0,15 g/L, Eisensulfat (FeSO₄) zu 0,02 g/L, Sojapepton zu 5 g/L, Cystein zu 2,0 g/L und Hefeextrakt zu 8,3 g/L.

## Revendications

1. Procédé de fermentation pour l'obtention de polysaccharide capsulaire de *Neisseria meningitidis* du sérogroupe A, comprenant :
a) l'inoculation de *Neisseria meningitidis A* dans un milieu de fermentation ayant un pH compris entre 7 et 7,5 et comprenant les composés suivants:
Dextrose de 8 g/L à 10 g/L, Chlorure de sodium de 5,6 g/L à 6 g/L, Sulfate dipotassique de 0,8 g/L à 1 g/L, Arginine de 0,2 g/L à 0,4 g/L, Sérine de 0,4 g/L à 0,6 g/L, Cystéine de 0,20 g/L à 0,25 g/L, Chlorure de magnésium (MgCl₂) de 0,18 g/L à 0,20 g/L, Chlorure de calcium (CaCl₂) de 0,020 g/L à 0,022 g/L, Sulfate ferreux (FeSO₄) à 0,002 g/L, Acide L-glutamique de 4,8 g/L à 5,2 g/L, Peptone de soja de 8,8 g/L à 9,2 g/L, Chlorure d'ammonium (NH₄Cl) de 0,44 g/L à 0,46 g/L, Hydrogéno-phosphate dipotassique (K₂HPO₄) de 3,8 g/L à 4,2 g/L et Extrait de levure de 5,30 g/L à 5,35 g/L ;
b) l'ajout, à partir de la phase log, d'une solution d'alimentation comprenant les composés suivants:
Dextrose de 70 g/L à 75 g/L, Glutamate monosodique de 37 g/L à 37,5 g/L, Arginine de 2,8 g/L à 3,2 g/L, Sérine de 2,8 g/L à 3,2 g/L, Chlorure de magnésium (MgCl₂) de 1,8 g/L à 2,2 g/L, Chlorure de calcium (CaCl₂) de 0,13 g/L à 0,15 g/L, Sulfate ferreux (FeSO₄) à 0,02 g/L, Peptone de soja de 4,8 g/L à 5,2 g/L, Cystéine de 1,8 g/L à 2,2 g/L et Extrait de levure de 8,2 g/L à 8,4 g/L ;
c) l'ajout d'un agent antimousse qui est un ester d'acide gras alcoxylé sur une base végétale en solution de 10% v/v à 20% v/v, à partir de 4 heures de fermentation jusqu'à 11 heures, où la concentration finale de Struktol **J673A®**dans le fermenteur est comprise entre 0,01% et 0,1% v/v ;
d) la récolte et la collecte de 2 g/L à 2,5 g/L de polysaccharide capsulaire de *Neisseria meningitidis A.*

2. Procédé selon la revendication 1, comprenant en outre, après ladite étape d), une étape de purification comprenant :
- l'ajout d'un mélange de désoxycholate à 1%, d'acétate de sodium à 6%, d'EDTA 2 mM et d'éthanol à 40% au polysaccharide capsulaire de *Neisseria meningitidis A* récolté, diafiltré 100KD ;
- l'agitation dudit mélange à 2―8°C pendant 3-4 h ;
- la centrifugation à 10 000 tours/min pendant 20 minutes dudit mélange et la diafiltration du surnageant collecté contre du Tris 25 mM avec une membrane à cassette 100KD ;
- la précipitation avec du cétyltriméthylammonium à 3% p/v pendant une nuit à 2-8°C sous agitation ;
- la collecte du culot et sa dissolution dans de l'éthanol à 96% avec du NaCl 0,05M à 2-8°C pendant 2 heures sous agitation ;
- la précipitation pendant 30 minutes et la redissolution du culot dans de l'éthanol à 45% avec du NaCl 0,4M pendant 1 h ;
- la collecte du surnageant et la filtration à travers un filtre à charbon ;
- la précipitation dans de l'éthanol à 96% pendant 1-2 h et la collecte du culot qui est du polysaccharide de *N. meningitidis* A purifié.

3. Procédé selon la revendication 1 ou 2, dans lequel ladite addition d'antimousse commence au début de la phase Log, à environ la 4^{ème} heure de fermentation, à un débit d'environ 20 ml/h, et ensuite, se poursuit à un débit d'environ 35-50 ml/h jusqu'à la fin de la phase Log, entre environ la 11^{ème} et la 14^{ème} heure de fermentation.

4. Procédé selon la revendication 2 ou 3, dans lequel ledit polysaccharide a un poids moléculaire moyen en poids de 800 à 1200 KDa, et ledit polysaccharide purifié contient moins de 0,3% p/p de protéines/peptides, moins de 0,07% p/p d'acides nucléiques et moins de 10EU/µg d'endotoxines.

5. Procédé selon la revendication 1, dans lequel ledit milieu de fermentation comprend les composés suivants: Dextrose à 10 g/L, Chlorure de sodium à 5,8 g/L, Sulfate dipotassique à 1 g/L, Arginine à 0,3 g/L, Sérine à 0,5 g/L, Cystéine à 0,23 g/L, Chlorure de magnésium (MgCl₂) à 0,19 g/L, Chlorure de calcium (CaCl₂) à 0,021 g/L, Sulfate ferreux (FeSO₄) à 0,002 g/L, Acide L-glutamique à 5 g/L, Peptone de soja à 9 g/L, Chlorure d'ammonium (NH₄Cl) à 0,45 g/L, Hydrogéno-phosphate dipotassique (K₂HPO₄) à 4 g/L et Extrait de levure à 5,34 g/L.

6. Procédé selon la revendication 1, dans lequel ladite solution d'alimentation comprend les composés suivants : Dextrose à 75 g/L, Glutamate monosodique à 37,5 g/L, Arginine à 3,0 g/L, Sérine à 3,2 g/L, Chlorure de magnésium (MgCl2) de 1,8 g/L à 2,2 g/L, Chlorure de calcium (CaCl₂) de 0,13 g/L à 0,15 g/L, Sulfate ferreux (FeSO₄) à 0,02 g/L, Peptone de soja à 5 g/L, Cystéine à 2,0 g/L et Extrait de levure à 8,3 g/L.
